# FASCICULE DE BREVET EUROPEEN

(11) **EP 3 416 707 B1**
(45) Date de publication et mention de la délivrance du brevet: **27.07.2022**
(21) Numéro de dépôt: 17706840.0
(22) Date de dépôt: 13.01.2017
(51) Int. Cl.: A61M 5/20, A61M 5/32

(54) **DISPOSITIF D'INJECTION D'UN PRODUIT LIQUIDE À ASSEMBLAGE SIMPLIFIÉ**
VORRICHTUNG ZUM EINSPRITZEN EINES FLÜSSIGEN PRODUKTS MIT VEREINFACHTER MONTAGE
DEVICE FOR INJECTING A LIQUID PRODUCT, HAVING SIMPLIFIED ASSEMBLY

(30) Priorité: 19.02.2016 FR 1651377
(43) Date de publication de la demande: 26.12.2018
(73) Titulaire: Nemera La Verpilliere, 38290 La Verpilliere (FR)
(72) Inventeur: DUGAND, Pascal, 38780 ESTRABLIN (FR); STAMP, Kevin, Sheffield S351AR (GB)
(74) Mandataire: LLR
(86) Numéro de dépôt international: PCT/FR2017/050077
(87) Numéro de publication internationale: WO 2017/140962

(56) Documents cités:
- EP-A2- 2 055 334
- WO-A1-2009/081133
- WO-A1-2011/012849
- DE-U1-202004 016 791
- FR-A1- 2 852 851

## Description

La présente invention concerne le domaine des dispositifs d'injection automatique de produit liquide, notamment pharmaceutique.

Un dispositif d'injection automatique est généralement un dispositif médical permettant l'administration automatique d'un médicament liquide nécessitant une injection. Ces dispositifs permettent en particulier que des personnes, par exemple atteintes d'arthrite rhumatoïde, de sclérose en plaque, de diabète ou subissant un choc anaphylactique en cas d'allergie, s'injectent elles-mêmes leur dose de médicament de manière autonome.

Sont notamment connus le document FR 2852851 A1, lequel divulgue un dispositif de protection d'aiguille, le document EP 2055334 A2, lequel divulgue un dispositif de seringue, le document WO 2009081133 A1, lequel divulgue un dispositif d'injection automatique, et le document WO 2011012849 A1, lequel divulgue un dispositif de seringue.

Un exemple de dispositif d'injection automatique est décrit dans le document US8734402. Le dispositif comprend une seringue d'injection qui contient le produit liquide à injecter et qui est munie d'une aiguille, et d'un support de seringue. Il suffit généralement d'appuyer brièvement le dispositif sur la peau du patient pour déclencher une pénétration de l'aiguille dans la peau, suivie d'une injection du produit liquide, puis de la rétractation de l'aiguille à l'intérieur du dispositif pour éviter de blesser une personne avec l'aiguille.

Plus précisément, le dispositif comprend un support de seringue configuré pour loger la seringue d'injection, de façon qu'elle soit montée fixe dans le support de seringue tout au long du fonctionnement du dispositif. Ce support de seringue est formé par deux demi-coques, semi-tubulaires, assemblées l'une à l'autre une fois la seringue d'injection disposée à l'intérieur, de façon à former une coque tubulaire autour de la seringue.

La seringue d'injection comprend une collerette à l'une de ses extrémités, et un capuchon de protection d'aiguille, par exemple un capuchon appelé RNS (pour "Rigid Needle Shield"), à son autre extrémité. Le diamètre du capuchon de protection est souvent supérieur à celui du corps de la seringue.

Le support de seringue dans lequel est logée la seringue a une forme générale de tube dont le diamètre est proche de celui de la partie centrale de la seringue, il n'est pas toujours possible d'insérer la seringue dans le support de seringue après que les deux demi-coques qui le composent aient été assemblées, car la collerette et le capuchon sont plus larges que la partie centrale de la seringue et donc plus large que le diamètre intérieur du support de seringue. Or généralement l'assemblage de la seringue d'injection dans le dispositif d'injection automatique est réalisé par un laboratoire pharmaceutique et non par le fabricant du dispositif d'injection automatique, de sorte que toute simplification de l'assemblage de la seringue d'injection dans le dispositif d'injection automatique évite que le laboratoire pharmaceutique ait besoin de s'équiper en appareillages complexes.

L'invention a notamment pour but de fournir un dispositif d'injection automatique dans lequel l'assemblage de la seringue d'injection dans le dispositif d'injection automatique est simplifié.

A cet effet, l'invention a pour objet un dispositif d'injection automatique tel que défini dans la revendication 1.

Ainsi, grâce au fait que la bague déformable peut être en position de passage du capuchon de protection, il est possible d'assembler la seringue d'injection portant le capuchon de protection dans la partie du dispositif d'injection en insérant la seringue d'injection axialement par une extrémité du support de seringue, le capuchon de protection étant inséré le premier. Il n'est donc plus nécessaire d'assembler le support de seringue après y avoir positionné la seringue d'injection et il est donc possible d'assembler les deux demi-coques composant le support de seringue avant d'y introduire la seringue d'injection ou d'utiliser un support de seringue qui n'est pas constitué de deux demi-coques.

En outre, comme la bague déformable en configuration d'immobilisation retient axialement la seringue d'injection dans le support de seringue, le positionnement et le maintien axial de la seringue d'injection dans le support de seringue n'ont pas besoin d'être assurés par la collerette de la seringue d'injection, de sorte que l'on évite de fragiliser la seringue d'injection au niveau de cette collerette. En effet, on sait que le ressort permettant d'assurer l'injection de produit peut être un ressort relativement puissant. Or, lorsque la seringue d'injection est tenue par sa collerette, cette dernière doit retenir la seringue d'injection lorsque le ressort exerce une pression sur la tige de piston pour réaliser l'injection et lorsque le déplacement du piston est limité par la contrepression du produit liquide à injecter contenu dans la seringue d'injection et les frottements entre le piston et l'intérieur du corps de la seringue d'injection. Cette pression est d'autant plus importante lorsque le produit liquide est visqueux. Aussi, la collerette est fortement sollicitée pendant l'injection, ce qui risque d'endommager ou casser la seringue d'injection, en particulier lorsque cette dernière est en verre. Comme la seringue d'injection est retenue ici par son extrémité distale et que les contraintes se traduisent donc par une compression concentrée sur l'extrémité distale de la seringue d'injection, l'extrémité proximale de la seringue d'injection, et donc la collerette, est épargnée. Notons que des matériaux tels que le verre sont généralement plus résistants en compression qu'en flexion. Il devient ainsi envisageable d'utiliser des ressorts particulièrement rigides, et donc d'injecter un produit présentant une viscosité plus élevée qu'auparavant. En particulier, il est possible d'utiliser un ressort d'injection présentant une force en position comprimée de 20 Newton, voire 50 Newton ou encore 80 Newton ou plus. On comprendra qu'il est également envisageable d'utiliser une seringue d'injection ne comportant pas de collerette ou encore que la seringue d'injection prenne la forme d'une cartouche de réception du produit liquide.

Selon d'autres caractéristiques optionnelles correspondant à différents modes de réalisation de la partie distale du dispositif d'injection automatique :
- la bague déformable est rappelée élastiquement vers sa configuration d'immobilisation, la bague déformable se déformant à l'encontre de sa force élastique de rappel depuis sa configuration d'immobilisation jusqu'à sa configuration de passage par déplacement radial centrifuge du secteur portant la butée axiale ;
- la saillie radiale de pilotage forme également un tenon de liaison avec le support de seringue en étant emboîté dans un étrier du support de seringue formant mortaise ;
- le support de seringue est déplaçable par rapport au manchon d'extrémité entre :
   - une position de montage du dispositif d'injection dans laquelle la saillie radiale de pilotage est alignée radialement avec une fenêtre de passage ménagée dans le manchon d'extrémité de façon à permettre le déplacement radial centrifuge du secteur portant la butée axiale vers la configuration de passage du capuchon de protection, et
   - une position active du dispositif d'injection dans laquelle la saillie radiale de pilotage coopère avec la surface complémentaire solidaire du manchon d'extrémité de façon à interdire le déplacement radial centrifuge du secteur portant la butée axiale vers la configuration de passage du capuchon de protection ;
- le secteur portant la butée axiale est muni d'au moins une fente axiale facilitant la déformation élastique de la bague déformable à rencontre de sa force élastique de rappel depuis sa configuration d'immobilisation jusqu'à sa configuration de passage par déplacement radial centrifuge du secteur portant la butée axiale ;
- la bague déformable est rappelée élastiquement vers sa configuration de passage, la bague déformable se déformant à l'encontre de sa force élastique de rappel de sa configuration de passage jusqu'à sa configuration d'immobilisation par déplacement radial centripète du secteur portant la butée axiale ;
- le support de seringue est déplaçable par rapport au manchon d'extrémité entre :
   - une position d'attente du dispositif d'injection dans laquelle la bague déformable est dans sa configuration de passage, et
   - une position active du dispositif d'injection dans laquelle la bague déformable est dans sa configuration d'immobilisation axiale de la seringue d'injection dans le support de seringue,
   la saillie radiale de pilotage coopérant, depuis la position d'attente jusqu'à la position active du dispositif d'injection, avec la surface complémentaire du manchon d'extrémité, cette surface complémentaire formant une rampe provoquant le déplacement radial centripète du secteur portant la butée axiale vers la configuration d'immobilisation de la bague déformable ;
- la bague déformable est essentiellement en matériau polymère.

L'invention sera mieux comprise à la lecture de la description qui va suivre donnée uniquement à titre d'exemples et faite en se référant aux dessins annexés, dans lesquels :
- la figure 1 est une vue en perspective avec une coupe axiale partielle d'un dispositif d'injection d'un produit liquide selon un premier mode de réalisation de l'invention ;
- les figures 2 et 3 sont des vues en perspective, suivant deux points de vue différents, d'une bague déformable élastiquement du dispositif d'injection illustré sur la figure 1 ;
- la figure 4 est une vue d'une partie du dispositif d'injection illustré sur la figure 1 en coupe partielle suivant un plan perpendiculaire au plan de coupe de la figure 1, la bague déformable étant dans une configuration de passage du capuchon ;
- les figures 5 et 6 sont des vues similaires à la figure 1, le capuchon amovible ayant été retiré, ces figures illustrant respectivement le dispositif d'injection dans des configurations avant et après insertion de l'aiguille de la seringue d'injection dans la peau d'un patient ;
- les figures 7 et 8 sont des vue en perspective d'une partie du dispositif d'injection de la figure 1, la bague déformable étant respectivement dans ses configurations de passage du capuchon et d'immobilisation axiale de la seringue ;
- les figures 9 et 10 sont des vues en perspective, suivant deux points de vue différents, d'une variante de réalisation de la bague déformable élastiquement illustrée sur les figures 2 et 3 ;
- la figure 11 est une vue en coupe dans un plan similaire à celui de la figure 4 d'un dispositif d'injection selon un deuxième mode de réalisation de l'invention, la bague déformable étant dans une configuration de passage du capuchon ;
- la figure 12 est une vue similaire à celle de la figure 11, dans laquelle le capuchon a été retiré et le dispositif d'injection est dans une configuration après insertion de l'aiguille de la seringue d'injection dans la peau d'un patient ;
- les figures 13 et 14 sont des vues en perspective, suivant deux points de vue différents, d'une bague déformable élastiquement du dispositif d'injection illustré sur la figure 11.

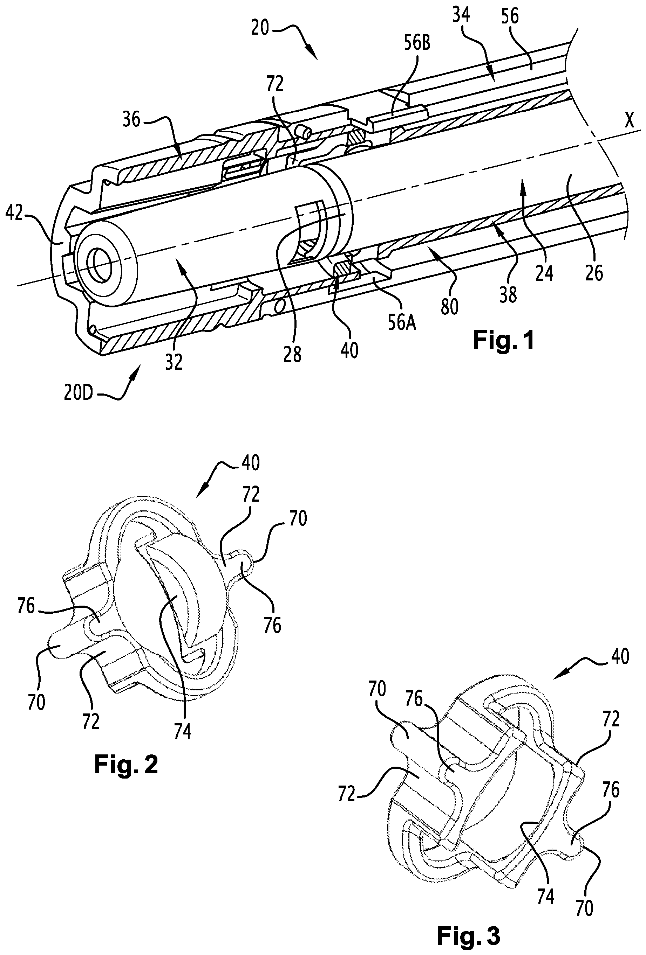

On a représenté sur les figures 1 à 10 un dispositif d'injection 20 d'un produit liquide, selon un premier mode de réalisation de l'invention. Plus précisément, le dispositif d'injection 20 est un dispositif médical permettant l'administration automatique d'un médicament liquide par injection.

Ainsi, le dispositif d'injection 20 sert à administrer un produit liquide à un patient, plus particulièrement un produit pharmaceutique administré par injection. Ce dispositif d'injection 20 a essentiellement une forme générale cylindrique d'axe X. Pour réaliser une injection, le patient saisit le dispositif d'injection 20 par une extrémité et applique l'autre extrémité de ce dispositif d'injection 20 contre sa peau. Une suite de mouvements de différents organes du dispositif d'injection 20 pilotés automatiquement entraine alors l'insertion d'une aiguille d'injection 22 d'une seringue d'injection 24 dans la peau du patient puis l'injection de son contenu à travers l'aiguille d'injection 22.

On qualifiera, dans la suite, de proximal un élément du dispositif d'injection 20 proche de la main du patient et de distal un élément du dispositif d'injection 20 éloigné de la main du patient. Par conséquent l'extrémité du dispositif d'injection 20 que le patient applique contre sa peau est une extrémité distale du dispositif d'injection 20.

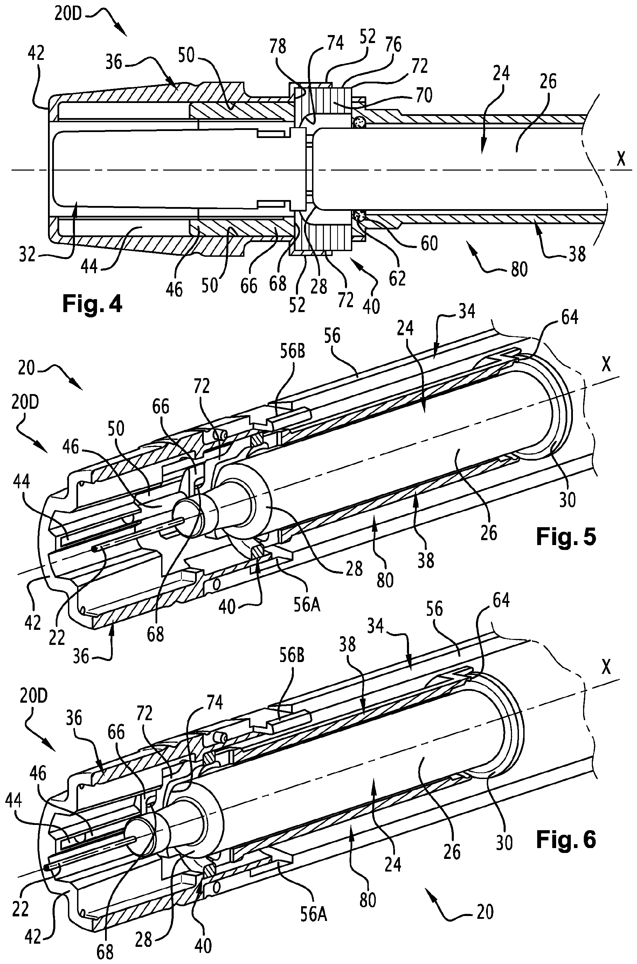

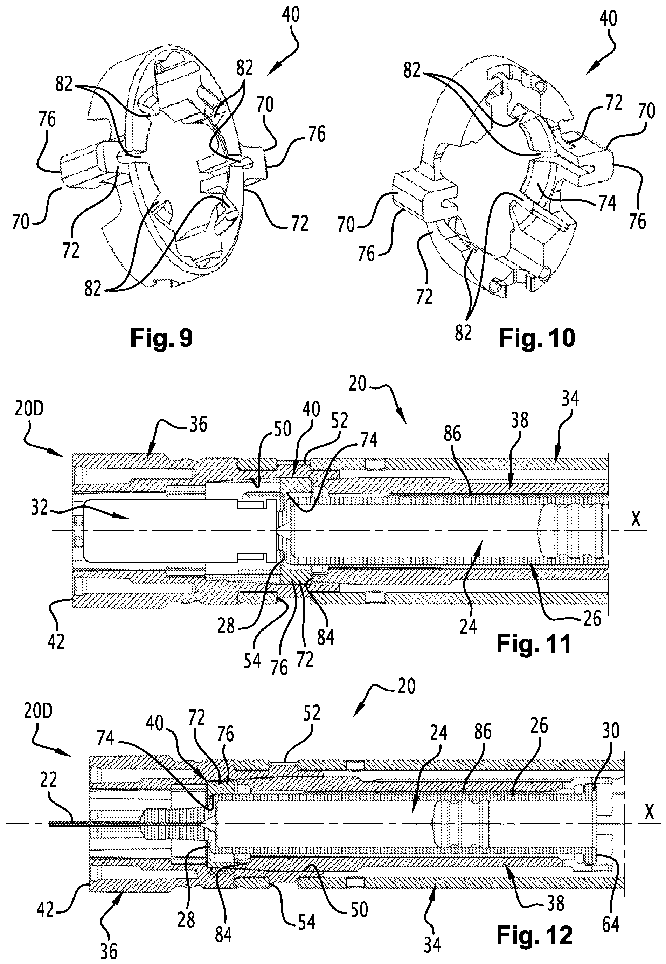

Comme on peut le voir, par exemple sur les figures 1 et 5, la seringue d'injection 24 comporte un corps de seringue 26 de forme globalement tubulaire autour de l'axe X. Le corps de seringue 26 porte l'aiguille d'injection 22 (visible sur la figure 5) à son extrémité distale. Le corps de seringue 26 comporte un épaulement distal 28 proche de l'aiguille d'injection 22. L'extrémité proximale du corps de seringue 26 comprend une collerette 30 (visible sur la figure 5). Un piston (non-visible sur les figures 1 à 10 du premier mode de réalisation mais visible sur les figures 11 et 12 du deuxième mode de réalisation) est monté coulissant dans le corps de seringue 26 et permet d'injecter un produit liquide contenu dans le corps de seringue 26 par l'aiguille d'injection 22 lorsqu'il se déplace vers l'extrémité distale du corps de seringue 26.

La seringue d'injection 24 est, dans cet exemple, une seringue pré-remplie en verre, à aiguille collée, ayant une contenance de 1 mL (millilitre). On notera que le corps de seringue 26 délimite un volume maximal de contenance de liquide, mais qu'il est envisageable de ne le remplir que partiellement, en avançant le piston vers l'extrémité distale du corps de seringue 26.

La figure 1 représente une partie distale 20D du dispositif d'injection 20 dans laquelle la seringue d'injection 24, munie d'un capuchon de protection 32, amovible et recouvrant l'aiguille d'injection 22, est logée. Cette partie distale 20D comprend différents organes transmettant des mouvements et des efforts produits par d'autres organes situés dans une partie proximale du dispositif d'injection 20 dont le fonctionnement est connu de l'homme du métier. Seul un élément 34 de la partie proximale du dispositif d'injection 20 est représenté sur les figures.

La partie distale 20D du dispositif d'injection 20 comprend un manchon d'extrémité 36 par rapport auquel un support de seringue 38 est monté coulissant (voir notamment les figures 4 à 6). Avant utilisation du dispositif d'injection 20, le coulissement du manchon d'extrémité 36 par rapport au support de seringue 38 est bloqué par un élément de blocage amovible, non représenté, logé dans le manchon d'extrémité 36 et contre lequel le support de seringue 38 vient en appui. La seringue d'injection 24 est logée dans le support de seringue 38. La partie distale 20D du dispositif d'injection 20 comprend aussi une bague déformable 40, représentée plus en détail sur les figures 2 et 3, liée cinématiquement au support de seringue 38. La bague déformable 40 est donc mobile par rapport au manchon d'extrémité 36. Cette bague 40 est déformable élastiquement entre une configuration destinée à l'immobilisation axiale de la seringue d'injection 24 dans le support de seringue 38 et une configuration destinée au passage du capuchon de protection 32 à travers la bague déformable 40.

Le manchon d'extrémité 36 est de forme essentiellement tubulaire et d'axe X. Après retrait de l'élément de blocage, sa surface la plus distale 42 est destinée à être mise en contact avec la peau du patient lors de l'injection. Deux gorges 44, rectilignes et de section au moins partiellement circulaire (voir figures 5 et 6), d'axes parallèles à l'axe X, sont ménagées dans le manchon d'extrémité 36. La surface intérieure du manchon d'extrémité 36 comprend deux parties, dites surfaces complémentaires 50 (voir figure 4), destinées à coopérer avec la bague déformable 40. Une fonction de ce manchon d'extrémité 36 est de protéger l'aiguille d'injection 22 contre tout contact involontaire avec un élément de son environnement après retrait du capuchon de protection 32 lorsque le dispositif d'injection 20 n'est pas activé, notamment pour éviter que l'utilisateur ne se pique involontairement avec l'aiguille d'injection 22.

L'élément 34 de la partie proximale représenté notamment sur les figures 1 et 4 est un organe de liaison 34 de la partie proximale avec la partie distale 20D du dispositif d'injection 20. Cet organe de liaison 34 est globalement tubulaire d'axe X et est assemblé au manchon d'extrémité 36 par des moyens comprenant au moins un ergot 52 (voir figure 4) porté par le manchon d'extrémité 36 destiné à coopérer avec une fenêtre de blocage de l'organe de liaison 34. Cette fenêtre de blocage n'est pas illustrée dans le cas du premier mode de réalisation mais porte la référence 54 sur les figures 11 et 12 illustrant le deuxième mode de réalisation.

L'organe de liaison 34 comprend deux demi-coques 56 assemblées l'une à l'autre par des moyens d'encliquetage comprenant divers encoches 56A et ergots 56B. Le manchon d'extrémité 36 et l'organe de liaison 34 (voir la figure 5), après qu'ils soient assemblés, restent fixes l'un par rapport à l'autre tout au long du fonctionnement du dispositif d'injection 20.

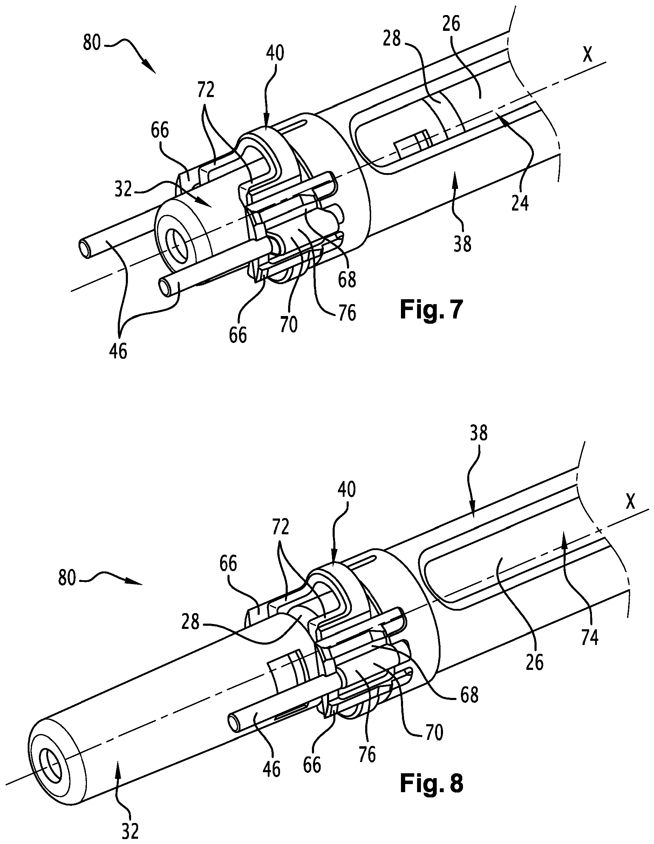

Le support de seringue 38 est un organe de forme tubulaire d'axe X et est ouvert à ses deux extrémités. L'extrémité distale 38D du support de seringue 38 est munie de deux tiges 58, cylindriques, représentées notamment sur les figures 4 et 5, destinées à coopérer avec les deux gorges 44 du manchon d'extrémité 36 pour guider en translation le support de seringue 38 dans son mouvement par rapport au manchon d'extrémité 36. La partie distale du support de seringue 38 est munie d'un logement distal 60 destiné à recevoir un anneau élastique 62 représenté sur la figure 4. Cet anneau élastique 62 permet de centrer le corps de seringue 26 dans le support de seringue 38 sans risque de casse de la seringue d'injection 24 et, lors de l'activation du dispositif d'injection 20, maintient axialement le corps de seringue 26 dans le support de seringue 38 de sorte que la translation du support de seringue 38 entraine la translation du corps de seringue 26 pour réaliser l'insertion de l'aiguille d'injection 22 dans le corps. Toutefois, cet anneau élastique 62 ne permet pas d'assurer le blocage axiale du corps de seringue 26 dans le support de seringue 38 au cours de l'injection. A son extrémité distale, le support de seringue 38 comporte un lamage proximal 64 (voir figures 5 et 6) destiné à recevoir la collerette 30 du corps de seringue 26. Il est à noter que le contact de ce lamage proximal 64 avec cette collerette 30 ne sert pas de butée dans l'immobilisation axiale de la seringue d'injection 24 dans le support de seringue 38, pendant le fonctionnement du dispositif d'injection 20. En effet lors du fonctionnement du dispositif d'injection 20, la collerette 30 de la seringue d'injection 24 ne subit pas les efforts axiaux liés à l'effort axial exercé sur le piston. Comme on peut le voir sur les figures 4 et 7, la partie distale du support de seringue 38 comporte deux étriers 66 formant deux mortaises 68 destinées à recevoir des tenons de liaison 70 de la bague déformable 40. La bague déformable 40 est ainsi liée cinématiquement au support de seringue 38. Le support de seringue 38 est assemblé avec le manchon d'extrémité 36 par des moyens non représentés sur les figures, connus de l'homme du métier, permettant le mouvement axial d'axe X du support de seringue 38 par rapport au manchon d'extrémité 36 après retrait de l'élément de blocage.

La bague déformable 40 est essentiellement en matériau polymère et est destinée à coopérer axialement avec la seringue d'injection 24. En se référant aux figures 2 et 3 on voit que la bague déformable 40 comprend au moins un secteur, de préférence deux secteurs 72 diamétralement opposés. Les secteurs 72 comportent des butées axiales 74 destinées chacune à coopérer avec l'épaulement distal 28 de la seringue d'injection 24. La bague déformable 40 comprend au moins une, de préférence deux saillies radiales diamétralement opposées dites de pilotage 76 de la bague déformable 40. Ces saillies radiales de pilotage 76 forment les tenons de liaison 70 avec le support de seringue 38 et sont portées par les secteurs 72 portant les butées axiales 74. Ces saillies radiales de pilotage 76 sont destinées à coopérer avec les surfaces complémentaires 50 portées par le manchon d'extrémité 36. Comme précisé plus haut, la bague déformable 40 peut se déformer entre une configuration destinée à l'immobilisation de la seringue d'injection 24 et une configuration destinée au passage du capuchon de protection 32. Le passage d'une configuration de la bague déformable 40 à l'autre se fait par déplacement radial des secteurs 72 portant les butées axiales 74. Pour ces secteurs 72, on parlera de configuration de passage du capuchon de protection 32 et de configuration d'immobilisation axiale de la seringue d'injection 24 lorsque les positions des secteurs 72 sont imposées par les configurations correspondantes de la bague déformable 40.

Lorsque la bague déformable 40 est montée dans le support de seringue 38, les deux saillies radiales de pilotage 76 sont emboîtées dans les mortaises 68 formées par les étriers 66 du support de seringue 38 (voir notamment la figure 8). Ainsi, la bague déformable 40 et le support de seringue 38 sont liés entre eux dans leur mouvement de translation selon l'axe X.

Lorsque la bague déformable 40 n'est pas contrainte, elle est rappelée élastiquement dans sa configuration d'immobilisation axiale de la seringue d'injection 24 dans le support de seringue 38. Dans cette configuration, l'espace entre les deux butées axiale 74 est suffisamment faible pour empêcher le passage de la seringue d'injection 24 à travers la bague déformable 40. Dans cette configuration également, les butées axiales 74 coopèrent avec l'épaulement distal 28 du corps de seringue 26 et reprennent les efforts axiaux dans le sens distal appliqués sur la seringue d'injection 24. La seringue d'injection 24 est donc immobilisée axialement de manière suffisante pour encaisser l'effort du ressort d'injection, même lors de l'injection de produits visqueux, sans que la seringue d'injection 24 ne se déplace axialement et sans que la collerette 30 n'ait à encaisser des efforts risquant de la détériorer.

Lorsque des efforts centrifuges sont appliqués sur les deux secteurs 72 de la bague déformable 40, la bague déformable 40 se déforme, à l'encontre de sa force de rappel, de telle sorte que les deux secteurs 72 portant les butées radiales de pilotage 76 se déplacent radialement depuis leur configuration d'immobilisation vers leur configuration de passage du capuchon de protection 32. Lorsque la bague déformable 40 est en configuration de passage du capuchon de protection 32, les butées axiales 74 sont plus espacées que lorsque la bague déformable 40 est en configuration d'immobilisation de façon à laisser libre un passage suffisant pour que le capuchon de protection 32 passe au travers de la bague déformable 40.

Pour monter le dispositif d'injection 20, on monte tout d'abord la seringue d'injection 24 dans la partie distale 20D alors que cette partie distale 20D est séparée de la partie proximale du dispositif d'injection 20.

Pour monter la seringue d'injection 24 dans la partie distale 20D du dispositif d'injection 20, cette seringue d'injection 24 est insérée dans le support de seringue 38 par l'extrémité proximale du support de seringue 38, le capuchon de protection 32 passant en premier à travers cette extrémité proximale. Lorsque le capuchon de protection 32 entre en contact avec la bague déformable 40, qui est alors en configuration d'immobilisation, la partie proximale du capuchon de protection 32 coopère avec la bague déformable 40 et applique des efforts centrifuges sur les deux secteurs 72 de la bague déformable 40. Ainsi la bague déformable 40 se déforme en configuration de passage du capuchon de protection 32 et permet au capuchon de protection 32 de passer. Cette déformation est possible car les saillies radiales de pilotage 76 de la bague déformable 40 sont alignées avec des fenêtres 78 de passage des saillies radiales de pilotage 76 (visibles sur la figure 4) portées par le manchon d'extrémité 36. Après le passage complet du capuchon de protection 32 à travers la bague déformable 40, le capuchon de protection 32 ne coopère plus avec la bague déformable 40, et celle-ci revient donc par rappel élastique dans sa configuration d'immobilisation.

Après avoir monté la seringue d'injection 20 dans la partie distale 20D on assemble cette partie distale 20D avec la partie proximale du dispositif d'injection 20. Pour effectuer cet assemblage, l'ensemble comprenant le manchon d'extrémité 36 et la seringue d'injection 24 est inséré dans la partie proximale du dispositif d'injection 20 de manière à ce que l'ergot 52 porté par le manchon d'extrémité 36 et la fenêtre de blocage 54 portée par l'organe de liaison 34 coopèrent et s'assemblent.

Avant activation, l'élément de blocage est retiré du manchon d'extrémité 36, ce qui libère la translation relative entre le support de seringue 38 et le manchon d'extrémité 36.

Lorsque le dispositif d'injection 20 est activé, une partie mobile 80 du dispositif d'injection 20, comportant la seringue d'injection 24, le support de seringue 38 et la bague déformable 40, se déplace par rapport au manchon d'extrémité 36 depuis une position d'attente ou de montage, vers une position active du dispositif d'injection 20. La position de montage correspond à la position d'attente avant que les parties distale 20D et proximales du dispositif d'injection 20 ne soient assemblées.

Lorsque la partie mobile 80 est en position de montage, les saillies radiales de pilotage 76 sont alignées radialement avec les fenêtres 78 de passage des saillies radiales de pilotage 76 ménagées dans le manchon d'extrémité 36 de façon à permettre le déplacement radial centrifuge des secteurs 72 portant les butées axiales 74 vers la configuration de passage du capuchon de protection 32. Dans cette position l'aiguille d'injection 22 est protégée par le manchon d'extrémité 36, c'est-à-dire que l'aiguille d'injection 22 ne dépasse pas axialement de ce manchon d'extrémité 36.

Lors de son déplacement par rapport au manchon d'extrémité 36 depuis la position d'attente vers la position active, la partie mobile 80 avance selon l'axe X dans le sens proximal vers distal. L'aiguille d'injection 22 avance donc vers la peau du patient puis la perce et pénètre ainsi dans le corps du patient. Lors de ce déplacement, les saillies radiales de pilotage 76 et les fenêtres 78 de passage des saillies radiales de pilotage 76 du manchon d'extrémité 36 se désalignent. Les saillies radiales de pilotage 76 coopèrent alors chacune avec la surface complémentaire correspondante 50 du manchon d'extrémité 36 de façon à interdire le déplacement radial centrifuge des secteurs 72 portant les butées axiales 74 vers la configuration de passage du capuchon de protection 32.

Ainsi lorsque le dispositif d'injection 20 est en position active, la bague déformable 40 ne peut plus se déformer vers sa configuration de passage, la seringue d'injection 24 étant alors immobilisée axialement par les butées axiales 74 des secteurs 72 de la bague déformable 40.

Dans une variante représentée sur les figures 9 et 10, la bague déformable 40 dispose d'au moins une fente axiale 82, de préférence de plusieurs fentes axiales 82. Ces fentes axiales 82 ont pour but de faciliter la déformation de la bague déformable 40 à l'encontre de sa force élastique de rappel depuis sa configuration d'immobilisation jusqu'à sa configuration de passage par déplacement radial centrifuge des secteurs 72 portant les butées axiales 74. Certains des secteurs de la bague déformable 40 délimités par les fentes axiales 82 peuvent être d'épaisseur faible pour présenter une résistance moindre à la déformation élastique et ainsi se déformer aisément lors du passage du capuchon de protection 32. D'autres secteurs de la bague déformable 40 peuvent avoir une épaisseur supérieure à celle des secteurs d'épaisseur faible, leur permettant de former des butées axiales 74 efficaces.

Dans ce premier mode de réalisation, la bague déformable 40 passe de sa configuration destinée au passage du capuchon de protection 32 à travers la bague déformable 40 à sa configuration destinée à l'immobilisation axiale de la seringue d'injection 24 dans le support de seringue 38 lors de l'assemblage du corps de seringue 26 portant le capuchon de protection 32 dans le support de seringue 38.

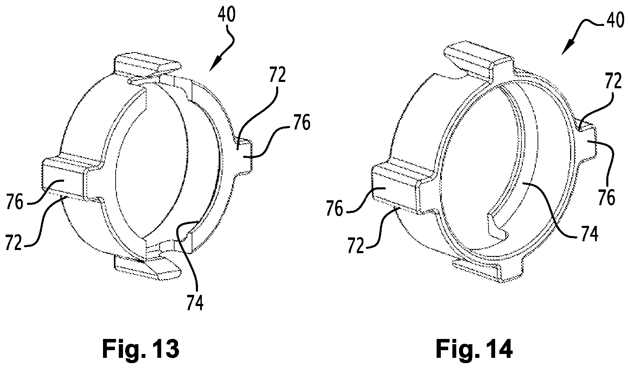

On décrira ci-dessous, en se référant aux figures 11 à 15, une partie distale 20D d'un dispositif d'injection 20 selon un deuxième mode de réalisation de l'invention. Dans ce cas, les éléments analogues à ceux des figures précédentes sont désignés par des références identiques.

Dans ce mode de réalisation, l'anneau élastique 62 est remplacé par des bandes 86 fixées contre la paroi intérieure du support de seringue 38, par exemple réalisées en TPE. Ces bandes 86 ont la même fonction que celle de l'anneau élastique 62.

Contrairement au mode de réalisation précédent, dans ce mode de réalisation, la bague déformable 40 est rappelée élastiquement vers sa configuration de passage du capuchon lorsqu'elle n'est pas contrainte. De plus, la bague déformable 40 est en simple appui contre l'extrémité distale du support de seringue 38. Cet appui permet à la bague déformable 40 d'être liée cinématiquement au support de seringue 38.

En effet, la bague déformable 40 se déforme depuis sa configuration de passage du capuchon de protection 32 vers sa configuration d'immobilisation axiale de la seringue d'injection 24 à l'encontre de sa force élastique de rappel par déplacement radial centripète des secteurs 72 portant les butées axiales 74.

La bague déformable 40 passe de sa configuration de passage du capuchon de protection 32 à sa configuration d'immobilisation de la seringue d'injection 24 par coopération avec les surfaces complémentaires 50. Ce changement de configuration se fait lorsque le support de seringue 38 coulisse dans le manchon d'extrémité 36 depuis une position d'attente vers une position active. Les surfaces complémentaires 50 portées par le manchon d'extrémité 36 forment des rampes 50 dont les parties distales sont plus proches de l'axe X que les parties proximales. Lorsque le support de seringue 38 est en position d'attente, les distances entre les rampes 50 et l'axe X sont suffisamment grandes pour que la bague déformable 40 ne se déforme pas et, ainsi, reste en configuration de passage. Lorsque le support de seringue 38 se déplace vers sa position active, les saillies radiales de pilotage 76 coopèrent avec les rampes 50, provoquant le déplacement radial centripète de chaque secteur 72 portant une butée axiale 74. La bague déformable 40 se déforme alors radialement vers sa configuration d'immobilisation axiale de la seringue d'injection 24 dans le support de seringue 38.

Ainsi, dans ce second mode de réalisation, la bague déformable 40 passe de sa configuration destinée au passage du capuchon de protection 32 à travers la bague déformable 40 à sa configuration destinée à l'immobilisation axiale de la seringue d'injection 24 dans le support de seringue 38 après l'activation du dispositif d'injection 20, lors de la phase d'insertion de l'aiguille d'injection 22 dans le corps.

Il est à noter que dans ce second mode de réalisation la bague déformable 40 n'est pas montée dans des mortaises portées par le support de seringue 38. La bague déformable 40 est simplement insérée dans le manchon d'extrémité 36 et est en contact avec un bord annulaire distal 84 du support de seringue 38 (voir figures 11 et 12). Lorsque la partie mobile 80 passe de sa position d'attente à sa position active, le support de seringue 38 pousse axialement la bague déformable 40. La bague déformable 40 et le support de seringue 38 sont alors liés entre eux en translation.

Dans ce deuxième mode de réalisation, l'assemblage des parties proximale et distale 20D du dispositif d'injection 20 et de la seringue d'injection 2 est similaire à celui décrit pour le premier mode de réalisation.

L'invention n'est pas limitée aux modes de réalisation présentés et d'autres modes de réalisation apparaîtront clairement à l'homme du métier. Il est notamment possible de ménager des fenêtres de passage des saillies radiales dans l'organe de liaison, de manière à ce que la bague déformable puisse toujours se déformer vers sa configuration de passage du capuchon lorsque le dispositif est complètement assemblé.

## Revendications

1. Dispositif d'injection (20) automatique d'un produit liquide, comprenant deux parties distale (20D) et proximale reliées entre elles, la partie distale (20D) comprenant :
- un manchon d'extrémité (36)
- un support de seringue (38) mobile par rapport au manchon d'extrémité (36), le support de seringue (38) étant destiné à porter une seringue d'injection (24) munie d'un capuchon de protection (32) amovible, la seringue d'injection (24) étant logée dans le support de seringue (38),
- une bague déformable (40), cette bague (40) étant déformable élastiquement entre une configuration destinée à l'immobilisation axiale de la seringue d'injection (24) dans le support de seringue (38) et une configuration destinée au passage du capuchon de protection (32) à travers la bague déformable (40),
la bague déformable (40) étant liée cinématiquement au support de seringue (38), la bague déformable (40) étant donc mobile par rapport au manchon d'extrémité (36), au moins un secteur (72) de la bague déformable (40), de préférence deux secteurs (72) diamétralement opposés de la bague déformable (40), comportant une butée axiale (74), la bague déformable (40) se déformant élastiquement entre ses configurations d'immobilisation et de passage par déplacement radial du secteur (72) portant la butée axiale (74),
**caractérisé en ce que** la butée axiale (74) est destinée à coopérer avec un épaulement distal (28) de la seringue d'injection (24) lorsque la bague déformable (40) est en configuration d'immobilisation axiale,
**et en ce que** la bague déformable (40) comprend au moins une saillie radiale (76) dite de pilotage de la bague déformable (40), de préférence deux saillies radiales de pilotage (76) diamétralement opposées, portée par le secteur (72) portant la butée axiale (74), cette saillie radiale de pilotage (76) étant destinée à coopérer avec une surface complémentaire (50) solidaire du manchon d'extrémité (36) selon la configuration de la bague déformable (40) que l'on veut imposer.

2. Dispositif d'injection (20) selon la revendication précédente, dans lequel la bague déformable (40) est rappelée élastiquement vers sa configuration d'immobilisation, la bague déformable (40) se déformant à l'encontre de sa force élastique de rappel depuis sa configuration d'immobilisation jusqu'à sa configuration de passage par déplacement radial centrifuge du secteur (72) portant la butée axiale (74).

3. Dispositif d'injection (20) selon la revendication précédente, dans lequel la saillie radiale de pilotage (76) forme également un tenon de liaison (70) avec le support de seringue (38) en étant emboîté dans un étrier (66) du support de seringue (38) formant mortaise (68).

4. Dispositif d'injection (20) selon la revendication 2, dans lequel le support de seringue (38) est déplaçable par rapport au manchon d'extrémité (36) entre :
- une position de montage du dispositif d'injection (20) dans laquelle la saillie radiale de pilotage (76) est alignée radialement avec une fenêtre (78) de passage ménagée dans le manchon d'extrémité (36) de façon à permettre le déplacement radial centrifuge du secteur (72) portant la butée axiale (74) vers la configuration de passage du capuchon de protection (32), et
- une position active du dispositif d'injection (20) dans laquelle la saillie radiale de pilotage (76) coopère avec la surface complémentaire (50) solidaire du manchon d'extrémité (36) de façon à interdire le déplacement radial centrifuge du secteur (72) portant la butée axiale (74) vers la configuration de passage du capuchon de protection (32).

5. Dispositif d'injection (20) selon la revendication 2, dans lequel le secteur (72) portant la butée axiale (74) est muni d'au moins une fente axiale (82) facilitant la déformation élastique de la bague déformable (40) à rencontre de sa force élastique de rappel depuis sa configuration d'immobilisation jusqu'à sa configuration de passage par déplacement radial centrifuge du secteur (72) portant la butée axiale (74).

6. Dispositif d'injection (20) selon la revendication 1, dans lequel la bague déformable (40) est rappelée élastiquement vers sa configuration de passage, la bague déformable (40) se déformant à rencontre de sa force élastique de rappel de sa configuration de passage jusqu'à sa configuration d'immobilisation par déplacement radial centripète du secteur (72) portant la butée axiale (74).

7. Dispositif d'injection (20) selon la revendication 6, dans lequel le support de seringue (38) est déplaçable par rapport au manchon d'extrémité (36) entre :
- une position d'attente du dispositif d'injection (20) dans laquelle la bague déformable (40) est dans sa configuration de passage, et
- une position active du dispositif d'injection (20) dans laquelle la bague déformable (40) est dans sa configuration d'immobilisation axiale de la seringue d'injection (20) dans le support de seringue (38),
la saillie radiale de pilotage (76) coopérant, depuis la position d'attente jusqu'à la position active du dispositif d'injection (20), avec la surface complémentaire (50) du manchon d'extrémité (36), cette surface complémentaire (50) formant une rampe (50) provoquant le déplacement radial centripète du secteur (72) portant la butée axiale (74) vers la configuration d'immobilisation de la bague déformable (40).

8. Dispositif d'injection (20) selon l'une quelconque des revendications précédentes, dans lequel la bague déformable (40) est essentiellement en matériau polymère.

## Patentansprüche

1. Automatische Injektionsvorrichtung (20) für ein flüssiges Produkt, die zwei miteinander verbundene distale (20D) und proximale Teile aufweist, wobei der distale Teil (20D) Folgendes aufweist:
- eine Endhülse (36)
- einen Spritzenhalter (38), der in Bezug auf die Endhülse (36) beweglich ist, wobei der Spritzenhalter (38) dazu bestimmt ist, eine Injektionsspritze (24) zu tragen, die mit einer abnehmbaren Schutzkappe (32) versehen ist, wobei die Injektionsspritze (24) in dem Spritzenhalter (38) untergebracht ist,
- einen verformbaren Ring (40), wobei dieser Ring (40) elastisch verformbar ist zwischen einer Konfiguration, die für die axiale Fixierung der Injektionsspritze (24) in dem Spritzenhalter (38) bestimmt ist, und einer Konfiguration, die für den Durchgang der Schutzkappe (32) durch den verformbaren Ring (40) bestimmt ist,
wobei der verformbare Ring (40) kinematisch mit dem Spritzenhalter (38) verbunden ist, der verformbare Ring (40) also relativ zur Endhülse (36) beweglich ist, wobei mindestens ein Sektor (72) des verformbaren Rings (40), vorzugsweise zwei diametral gegenüberliegende Sektoren (72) des verformbaren Rings (40), einen axialen Anschlag (74) aufweist, wobei sich der verformbare Ring (40) zwischen seinen Fixier- und Durchgangskonfigurationen durch radiale Verschiebung des Sektors (72), der den axialen Anschlag (74) trägt, elastisch verformt,
**dadurch gekennzeichnet, dass** der axiale Anschlag (74) dazu bestimmt ist, mit einer distalen Schulter (28) der Injektionsspritze (24) zusammenzuwirken, wenn sich der verformbare Ring (40) in der axialen Immobilisierungskonfiguration befindet,
und dadurch, dass der verformbare Ring (40) mindestens einen radialen Vorsprung (76) aufweist, der als Steuerung des verformbaren Rings (40) bezeichnet wird, vorzugsweise zwei diametral gegenüberliegende radiale Steuervorsprünge (76), die von dem Sektor (72) getragen werden, der den axialen Anschlag (74) trägt, wobei dieser radiale Steuervorsprung (76) dazu bestimmt ist, mit einer komplementären Fläche (50) zusammenzuwirken, die fest mit der Endhülse (36) verbunden ist, je nach der Konfiguration des verformbaren Rings (40), die man herbeiführen möchte.

2. Injektionsvorrichtung (20) nach dem vorhergehenden Anspruch, bei der der verformbare Ring (40) elastisch in Richtung seiner Fixierkonfiguration zurückgestellt wird, wobei sich der verformbare Ring (40) entgegen seiner elastischen Rückstellkraft von seiner Fixierkonfiguration in seine Durchgangskonfiguration durch zentrifugale Radialverschiebung des den axialen Anschlag (74) tragenden Sektors (72) verformt.

3. Injektionsvorrichtung (20) nach dem vorhergehenden Anspruch, bei der der radiale Steuervorsprung (76) auch einen Verbindungszapfen (70) mit dem Spritzenhalter (38) bildet, indem er in einen Bügel (66) des Spritzenhalters (38), der eine Zapfstelle (68) bildet, eingesteckt wird.

4. Injektionsvorrichtung (20) nach Anspruch 2, bei der der Spritzenhalter (38) relativ zur Endhülse (36) verschiebbar ist zwischen:
- einer Montageposition der Injektionsvorrichtung (20), in der der radiale Steuervorsprung (76) radial mit einem in der Endhülse (36) ausgebildeten Durchgangsfenster (78) ausgerichtet ist, um die zentrifugale Radialverschiebung des den axialen Anschlag (74) tragenden Sektors (72) in Richtung der Durchgangskonfiguration der Schutzkappe (32) zu ermöglichen, und
- einer aktiven Position der Injektionsvorrichtung (20), in der der radiale Steuervorsprung (76) mit der komplementären Fläche (50) zusammenwirkt, die fest mit der Endhülse (36) verbunden ist, um die zentrifugale Radialverschiebung des Sektors (72), der den axialen Anschlag (74) trägt, in Richtung der Konfiguration des Durchgangs der Schutzkappe (32) zu unterbinden.

5. Injektionsvorrichtung (20) nach Anspruch 2, bei der der den axialen Anschlag (74) tragende Sektor (72) mit mindestens einem axialen Schlitz (82) versehen ist, der die elastische Verformung des verformbaren Rings (40) gegen seine elastische Rückstellkraft von seiner Fixierkonfiguration in seine Durchgangskonfiguration durch zentrifugale Radialverschiebung des den axialen Anschlag (74) tragenden Sektors (72) erleichtert.

6. Injektionsvorrichtung (20) nach Anspruch 1, wobei der verformbare Ring (40) elastisch in Richtung seiner Durchgangskonfiguration rückgestellt ist, wobei sich der verformbare Ring (40) entgegen seiner elastischen Rückstellkraft aus seiner Durchgangskonfiguration in seine Fixierkonfiguration durch zentripetale Radialverschiebung des Sektors (72), der den axialen Anschlag (74) trägt, verformt.

7. Injektionsvorrichtung (20) nach Anspruch 6, wobei der Spritzenhalter (38) relativ zur Endhülse (36) verschiebbar ist zwischen:
- einer Bereitschaftsposition der Injektionsvorrichtung (20), in der sich der verformbare Ring (40) in seiner Durchgangskonfiguration befindet, und
- einer aktiven Position der Injektionsvorrichtung (20), in der sich der verformbare Ring (40) in seiner Konfiguration zur axialen Fixierung der Injektionsspritze (20) in dem Spritzenhalter (38) befindet,
wobei der radiale Steuervorsprung (76) von der Bereitschaftsstellung bis zur aktiven Stellung der Injektionsvorrichtung (20) mit der komplementären Fläche (50) der Endhülse (36) zusammenwirkt, wobei diese komplementäre Fläche (50) eine Rampe (50) bildet, die die zentripetale Radialverschiebung des Sektors (72), der den axialen Anschlag (74) trägt, in Richtung der Fixierkonfiguration des verformbaren Rings (40) bewirkt.

8. Injektionsvorrichtung (20) nach einem der vorhergehenden Ansprüche, wobei der verformbare Ring (40) im Wesentlichen aus einem polymeren Material besteht.

## Claims

1. A liquid product automatic injection device (20), comprising interconnected distal part (20D) and proximal part, the distal part (20D) comprising:
- an end sleeve (36)
- a syringe support (38) mobile relative to the end sleeve (36), the syringe support (38) being intended to carry an injection syringe (24) fitted with a removable protection cap (32), the injection syringe (24) being housed in the syringe support (38),
- a deformable ring (40), this ring (40) being elastically deformable between an immobilization configuration intended for the axial immobilization of the injection syringe (24) in the syringe support (38) and a passage configuration intended for the passage of the protection cap (32) through the deformable ring (40),
the deformable ring (40) being kinematically connected to the syringe support (38), the deformable ring 40 being therefore mobile relative to the end sleeve (36), at least one sector (72) of the deformable ring (40), preferably two diametrically opposite sectors (72) of the deformable ring (40), including an axial abutment (74), the deformable ring (40) being deformed axially between its immobilization and passage configurations by radial movement of the sector (72) carrying the axial abutment (74),
**characterized in that** the axial abutment (74) is intended to cooperate with a distal shoulder (28) of the injection syringe (24) when the deformable ring (40) is in the axial immobilization configuration,
**and in that** the deformable ring (40) comprises at least one steering radial projection (76) for steering the deformable ring (40), preferably two diametrically opposite steering radial projections (76), carried by the sector (72) carrying the axial abutment (74), this steering radial projection (76) being intended to cooperate with a complementary surface (50) integral with the end sleeve (36) according to the configuration to be imposed on the deformable ring (40).

2. The injection device (20) according to the preceding claim, wherein the deformable ring (40) is urged elastically toward its immobilization configuration, the deformable ring (40) being deformed against its elastic return force from its immobilization configuration to its passage configuration by centrifugal radial movement of the sector (72) carrying the axial abutment (74).

3. The injection device (20) according to the preceding claim, wherein the steering radial projection (76) also forms a tenon (70) for connection with the syringe support (38) by being nested in a bracket (66) of the syringe support (38) forming a mortise (68).

4. The injection device (20) according to claim 2, wherein the syringe support (38) is movable relative to the end sleeve (36) between:
- a position for mounting the injection device (20) in which the steering radial projection (76) is radially aligned with a passage window (78) formed in the end sleeve (36) so as to allow the centrifugal radial movement of the sector (72) carrying the axial abutment (74) toward the passage configuration of the protection cap (32), and
- an active position of the injection device (20) in which the steering radial projection (76) cooperates with the complementary surface (50) integral with the end sleeve (36) so as to prevent the centrifugal radial movement of the sector (72) carrying the axial abutment (74) toward the passage configuration of the protection cap (32).

5. The injection device (20) according to claim 2, wherein the sector (72) carrying the axial abutment (74) is provided with at least one axial slot (82) facilitating the elastic deformation of the deformable ring (40) against its elastic return force from its immobilization configuration to its passage configuration by centrifugal radial movement of the sector (72) carrying the axial abutment (74).

6. The injection device (20) according to claim 1, wherein the deformable ring (40) is urged elastically toward its passage configuration, the deformable ring (40) being deformed against its elastic return force from its passage configuration to its immobilization configuration by centripetal radial movement of the sector (72) carrying the axial abutment (74).

7. The injection device (20) according to claim 6, wherein the syringe support (38) is movable relative to the end sleeve (36) between:
- a waiting position of the injection device (20) in which the deformable ring (40) is in its passage configuration, and
- an active position of the injection device (20) in which the deformable ring (40) is in its axial immobilization configuration of the injection syringe (20) in the syringe support (38),
the steering radial projection (76) cooperating, from the waiting position to the active position of the injection device (20), with the complementary surface (50) of the end sleeve (36), this complementary surface (50) forming a ramp (50) causing the centripetal radial movement of the sector (72) carrying the axial abutment (74) toward the immobilization configuration of the deformable ring (40).

8. The injection device (20) according to any one of the preceding claims, wherein the deformable ring (40) is essentially made of polymer material.
